(19) Europäisches Patentamt
European Patent Office
Office européen des brevets

(11) Veröffentlichungsnummer: **0 188 007 B1**

(12) **EUROPÄISCHE PATENTSCHRIFT**

(45) Veröffentlichungstag der Patentschrift: **19.08.92**

(51) Int. Cl.5: **C07D 213/73**, //C07D213/75

(21) Anmeldenummer: **85116668.6**

(22) Anmeldetag: **31.12.85**

(54) **Verfahren zur Herstellung von 2-Nitro-3-aminopyridin sowie der dabei als Zwischenprodukt auftretende N,N'-Di-(2-nitro-3-pyridyl)Harnstoff.**

(30) Priorität: **12.01.85 DE 3500910**

(43) Veröffentlichungstag der Anmeldung:
**23.07.86 Patentblatt 86/30**

(45) Bekanntmachung des Hinweises auf die Patenterteilung:
**19.08.92 Patentblatt 92/34**

(84) Benannte Vertragsstaaten:
**BE CH DE FR GB IT LI NL SE**

(56) Entgegenhaltungen:
**DE-A- 2 165 757**

**CHEMICAL ABSTRACTS, Band 75, 1971, Seite 272, Zusammenfassung Nr. 98023f, Columbus, Ohio, US; Z. CSUROS et al.: "Thermal dissociation of urea derivatives"**

**JOURNAL OF THE CHEMICAL SOCIETY, 1957, Seiten 442-446, London, GB; J.W. CLARK-LEWIS et al.: "Methylation of 3-aminopyridines and preparation of 2-amino-3-methylaminopyridine and 2:3-diaminopyridine"**

(73) Patentinhaber: **HOECHST AKTIENGESELLSCHAFT**
**Postfach 80 03 20**
**W-6230 Frankfurt am Main 80(DE)**

(72) Erfinder: **Planker, Siegfried, Dr.**
**Kastanienweg 17**
**W-6240 Königstein/Taunus(DE)**
Erfinder: **Warning, Klaus, Dr.**
**Kreuzheck 6**
**W-6239 Eppstein/Taunus(DE)**
Erfinder: **Herbst, Horst, Dr.**
**Luisenstrasse 25**
**W-6233 Kelkheim (Taunus)(DE)**
Erfinder: **Schaeffer, Georg, Dr.**
**Herderstrasse 58**
**W-6238 Hofheim am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 188 007 B1

## Beschreibung

2-Nitro-3-aminopyridin ist die Verbindung der Formel

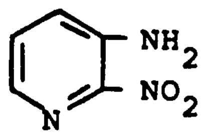

Sie ist hauptsächlich ein wertvolles Zwischenprodukt auf verschiedenen Sachgebieten wie dem Pharma-, Farbstoff- und Pflanzenschutzsektor. So werden z.B. durch Umsetzung des 2-Nitro-3-aminopyridins mit Aminoalkylhalogeniden 2-Nitro-3-aminoalkylamino-pyridine erhalten, die auf dem Pharmasektor zur Bekämpfung von Amöbeninfektionen einsetzbar sind (DE-A-23 34 401).

Durch direkte Nitrierung des einfach - z.B. durch Hofmann' schen Abbau von Nikotinsäureamid - zugänglichen und auch im Handel verfügbaren 3-Aminopyridins ist das 2-Nitro-3-aminopyridin nicht oder allenfalls in Spuren erhältlich, was wegen der bekannten Empfindlichkeit der (ungeschützten) Aminogruppe gegenüber - auch oxidativ wirkenden - Nitrierungsmitteln kaum verwunderlich ist.

Wenn man daher - gemäß dem Artikel von J. W. Clark-Lewis und M.J. Thompson in J. Chem. Soc. (London) 1957, S. 442-446, insbesondere S. 445/46 - von 3-Aminopyridin ausgeht, dessen Aminogruppe durch die Ethoxycarbonylgruppe geschützt ist, soll die Nitrierung mit einer bis zu 65 %igen Ausbeute gelingen. In dem Artikel wird allerdings ausdrücklich betont, daß die Nitrierung nur in kleinen Ansätzen durchführbar ist, weil die Reaktion nur schwer kontrollierbar verläuft. Das hierbei gebildete 2-Nitro-3-ethoxycarbonylamino-pyridin liefert durch alkalische Hydrolyse dann das gewünschte 2-Nitro-3-aminopyridin; die Ausbeuteangabe für die Hydrolysestufe beträgt 86 %. Bezogen auf das 3-Ethoxycarbonylaminopyridin liegt die Ausbeute somit bei etwa 56 %.

Die dieser Umsetzung zugrundeliegenden Reaktionsgleichungen sind (schematisch):

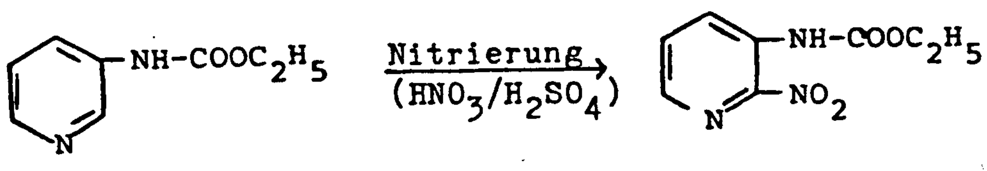


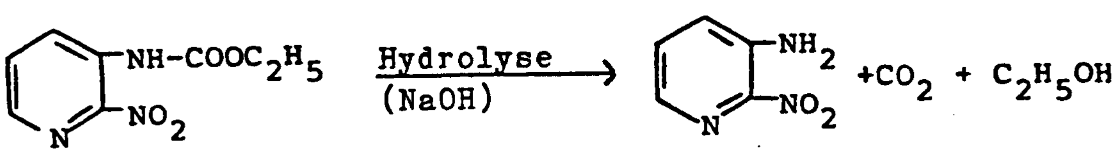


Das Ausgangsprodukt für diese Umsetzung (3-Ethoxycarbonylaminopyridin) wird nach der vorerwähnten Literaturstelle durch die nachstehende - nicht ganz unaufwendige - Reaktionsfolge, ausgehend von Nikotinsäure, erhalten:

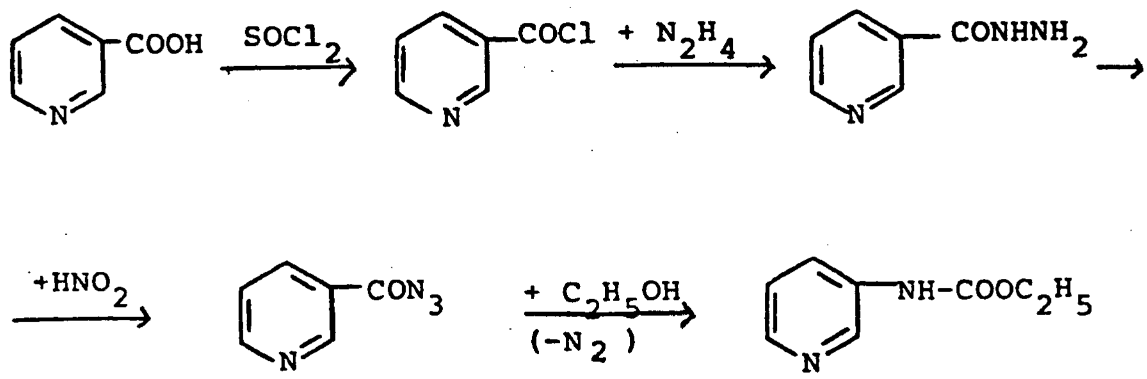


Die Einführung anderer Schutzgruppen für die Aminogruppe hat sich - wie eigene Versuche ergeben haben - als ungeeignet erwiesen, da z.T. bei der Nitrierung ebenfalls (wie bei der Reaktion gemäß J.W. Clark Lewis und M.J. Thompson a.a.O.) Zersetzung erfolgt; z.T. tritt die Nitrogruppe auch an einer anderen als der gewünschten (2-) Stellung des Pyridinrings ein. Die getesteten Schutzgruppen und Klassen von Schutzgruppen waren:

$-COCH_3$,
$-COC_6H_5$,
$-CO-C_6H_4-NO_2$,
$-COOCH_3$,
$-COOC_6H_5$,

$-CONR^1_2$ ($R^1$ = $C_1$-$C_4$-Alkyl),
$-CONHR^1$ ( " ),
$-SO_2R^2$ ( " , oder ggf. subst. Phenyl).
$-CH_2-NH-$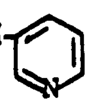

In der rein aromatischen (nicht heterocyclischen) Reihe ist auch die Nitrierung eines Anilinderivates (2-Methoxyanilin) bekannt geworden, welches zum Schutz der Aminogruppe vor der Nitrierung in ein Harnstoffderivat (Bis-2-methoxyphenyl-harnstoff) übergeführt wurde. Die Nitrierung erfolgte hier in 4-Stellung. Die Reaktion ist veröffentlicht in Chemical Abstracts, Vol. 49 (1955), 5341i bis 5342b; die Veröffentlichung stellt das Referat einer Arbeit von D.F. Kutepov und Z.G. Vukolova, Zh.Obsc. Khim. 24, 698-792 (1954) dar.

Die maßgebliche Reaktionsfolge ist hier (schematisch):

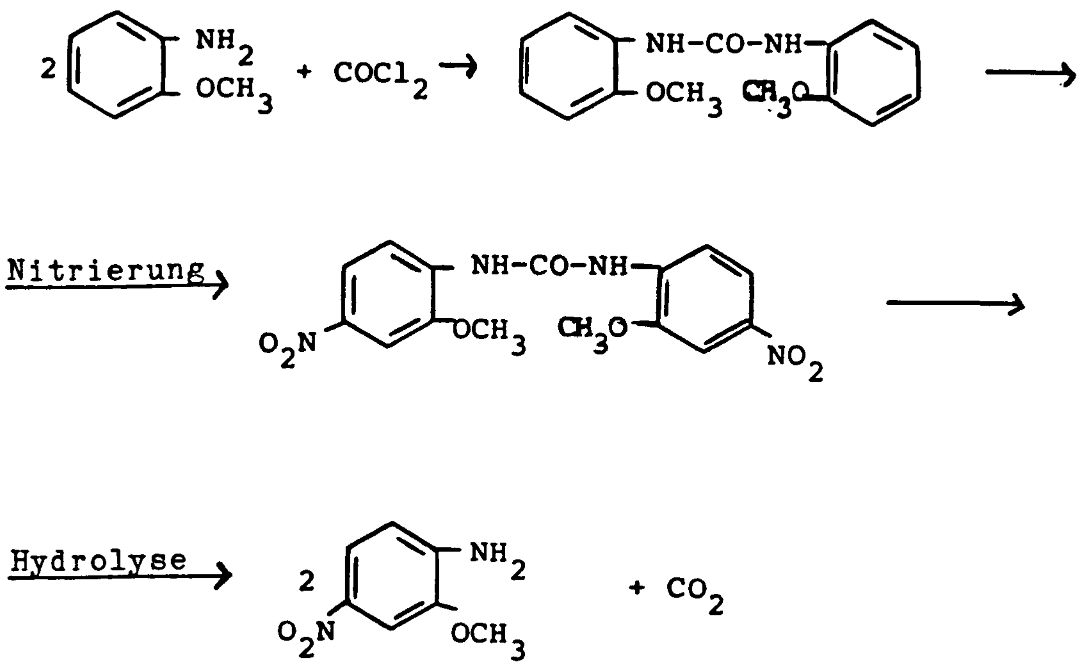


In dem Bestreben, ein verbessertes Verfahren zur Herstellung von 2-Nitro-3-aminopyridin bereitzustellen, wurde nun gefunden, daß dieses Ziel durch Nitrierung und hydrolytische Spaltung von N,N'-Di-(3-pyridyl)-harnstoff erreicht wird.

Erfindungsgegenstand ist daher ein Verfahren zur Herstellung von 2-Nitro-3-aminopyridin durch Nitrierung von 3-Aminopyridin, dessen $NH_2$-Gruppe mit einer Schutzgruppe versehen ist, und anschließende hydrolytische Abspaltung der Schutzgruppe,
das dadurch gekennzeichnet ist, daß man als 3-Aminopyridin, dessen $NH_2$-Gruppe mit einer Schutzgruppe versehen ist, N,N'-Di-(3-pyridyl)-harnstoff verwendet.

Dem Verfahren liegen folgende Reaktionsgleichungen zugrunde:

(1) 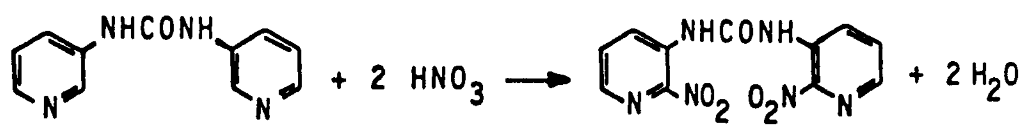

(2) 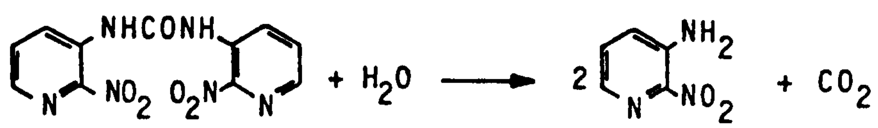

Die Nitrierung des N,N'-Di-(3-pyridyl)-harnstoffs erfolgt gefahrlos, sehr gut kontrollierbar und außerordentlich selektiv nur in (der gewünschten) 2-Stellung des Pyridinrings mit hohen Ausbeuten (durchweg über 90 % d.Th.). Die Hydrolyse des N,N'-Di-(2-nitro-3-pyridyl)-harnstoffs erfolgt ebenfalls mit Ausbeuten von durchweg über 90 % d.Th., so daß die Gesamtausbeute des Verfahrens üblicherweise über 80 % d.Th., bezogen auf die Ausgangsverbindung N,N'-Di-(3-pyridyl)-harnstoff beträgt.

Insbesondere der außerordentlich selektive und glatte Verlauf der Nitrierungsstufe ist sehr überraschend, weil nach den vorerwähnten eigenen Versuchen im Falle der Einführung einer ganzen Reihe anderer Schutzgruppen für die Aminogruppe des 3-Aminopyridins die Nitrierung nicht - jedenfalls nicht in der gewünschten Weise - gelingt
und weil bei der Nitrierung der mit N,N'-Di-(3-pyridyl)-harnstoff strukturell ähnlichen rein aromatischen Verbindung Bis-(2-methoxyphenyl)-harnstoff die Nitrogruppe nur in 4-Stellung (zur Aminogruppe) eintritt (vgl. das vorerwähnte Referat in Chemical Abstracts).

Die Ausgangsverbindung für das erfindungsgemäße Verfahren - N,N'-Di-(3-pyridyl)-harnstoff - wird auf

übliche Weise hergestellt, z.B. durch Umsetzung von 3-Aminopyridin mit Phosgen $COCl_2$ analog der Umsetzung von 2-Aminopyridin mit Phosgen (Beilstein H 22, 330).

Die Nitrierung des N,N'-Di-(3-pyridyl)-harnstoffs wird auf die für derartige Nitrierungsreaktionen übliche Weise mittels Salpetersäure, zweckmäßig in Gegenwart von Schwefelsäure, durchgeführt. Man kann beispielsweise den N,N'-Di-(3-pyridyl)-harnstoff in Schwefelsäure oder in Oleum vorlegen und dann mit Salpetersäure oder einem Gemisch aus Salpetersäure und Schwefelsäure ("Nitriersäure") versetzen, oder man kann etwa auch festen N,N'-Di-(3-pyridyl)-harnstoff in eine vorgelegte Mischung aus Salpetersäure und Schwefelsäure eintragen.

Die Reaktionstemperatur ist hierbei nicht besonders kritisch, doch sollte aus Gründen der Verfahrenssicherheit sowie der Produktqualität eine Temperatur von etwa 90 °C nicht wesentlich überschritten werden. Vorzugsweise wird die Reaktion bei etwa 50 - 70 °C durchgeführt.

Die Komponenten N,N'-Di-(3-pyridyl)-harnstoff und $HNO_3$ werden vorzugsweise in einem Molverhältnis (Pyridinring zu $HNO_3$) von 1:etwa 1-2, insbesondere 1:etwa 1,2-1,4, eingesetzt.

Die Isolierung des bei der Nitrierung gebildeten N,N'-Di-(2-nitro-3-pyridyl)-harnstoffs kann in üblicher Weise z.B. dadurch erfolgen, daß man die Reaktionsmischung nach beendeter Reaktion mit Wasser versetzt und dadurch das gewünschte Produkt in fester Form abscheidet, so daß es z.B. durch Filtration oder Zentrifugieren in reiner Form gewonnen werden kann.

Der N,N'-Di-(2-nitro-3-pyridyl)-harnstoff ist eine neue Verbindung.

Die Hydrolyse dieser Verbindung kann grundsätzlich in überschüssigem Wasser vorgenommen werden; bevorzugt ist jedoch der Zusatz von polaren Lösungsmitteln, insbesondere von Alkoholen. Zur Beschleunigung der Reaktion können, wie bei der Hydrolyse von Harnstoffderivaten an sich bekannt, Säuren oder Basen zugegeben werden. Eine bevorzugte Durchführungsart der Hydrolyse ist im vorliegenden Fall die Durchführung in einem wäßrigen $C_1$-$C_4$-Alkohol (Methanol, Ethanol, Isopropanol etc.) mittels anorganischer Basen (NaOH, KOH, $NH_3$ etc.), weil dann das bei der Hydrolyse gebildete $CO_2$ gleich als Carbonat oder Hydrogencarbonat der jeweiligen Base gebunden wird.

Beispielsweise wird zu einer Suspension von N,N'-Di-(2-nitro-3-pyridyl)-harnstoff in einem niederen Alkohol wie Methanol oder Ethanol wäßrige Natronlauge zudosiert, wobei das Molverhältnis Harnstoffderivat:NaOH vorzugsweise etwa 1:1-3, insbesondere etwa 1:1,5-2 beträgt. Gegen Ende der Reaktion kristallisiert das 2-Nitro-3-aminopyridin aus und kann in reiner Form isoliert werden.

Die folgenden Beispiele sollen nun der weiteren Erläuterung der Erfindung dienen. Auf die Erfindungsbeispiele (A) folgt noch eine Reihe von Vergleichsbeispielen (B), welche zeigen, daß die Nitrierung von 3-Aminopyridin, dessen Aminogruppe durch andere Schutzgruppen substituiert ist, nicht zu dem gewünschten Ziel (in kontrollierbarer Reaktion Nitrierung in 2-Stellung mit guten Ausbeuten) führt.

## A) Erfindungsbeispiele

### Beispiel 1

a) Nitrierung:

In einem 1,5 1-Rührapparat wurden 400 g 10 %iges Oleum und 100 g (0,47 mol) N,N'-Di-(3-pyridyl)-harnstoff vorgelegt und innerhalb von 1,5 Stunden bei einer Reaktionstemperatur von 60 °C mit 238 g Nitriersäure der Zusammensetzung 32 % $HNO_3$, 68 % $H_2SO_4$ versetzt. Anschließend wurde 3 Stunden bei dieser Temperatur nachgerührt, auf Raumtemperatur abgekühlt und mit 780 ml Wasser verdünnt. Die Suspension wurde bei 20 °C nachgerührt; anschließend wurde das Reaktionsprodukt abgenutscht und neutral gewaschen.

Ausbeute: 133 g N,N'-Di-(2-nitro-3-pyridyl)-harnstoff ≙ 93 % d.Th. Schmelzpunkt: 230 - 233 °C unter Zersetzung. Die NMR- und IR-Spektren waren in Übereinstimmung mit der genannten Struktur.

b) Hydrolyse:

Zur Hydrolyse wurden in einem 1,5 1-Rührapparat 415 g Ethanol vorgelegt und 133 g (0,44 mol) N,N'-Di-(2-nitropyridyl)-harnstoff eingetragen. Die Suspension wurde auf 78 °C erhitzt und mit 300 g 10 %iger Natronlauge versetzt. Während der Reaktion fiel das 2-Nitro-3-aminopyridin teilweise aus. Zur vollständigen Fällung des Reaktionsproduktes wurde nach Reaktionsende mit ca. 500 ml Wasser verdünnt, auf 0 bis +5 °C abgekühlt, die Suspension abgenutscht und das Reaktionsprodukt neutral gewaschen.

Ausbeute: 109,6 g 2-Nitro-3-aminopyridin ≙ 90,4 % d.Th., bezogen auf N,N'-Di-(2-nitro-3-pyridyl)-harnstoff. Schmelzpunkt: 199 - 200 °C.

Beispiel 2

In einem 1,5 l-Rührapparat wurden 400 g 10 %iges Oleum und 238 g Nitriersäure der Zusammensetzung 32 % $HNO_3$ und 68 % $H_2SO_4$ vorgelegt und innerhalb von 1,5 Stunden 100 g N,N'-Di-(3-pyridyl)-harnstoff bei einer Reaktionstemperatur von 60° C eingetragen.

Aufarbeitung und Verseifung zum 2-Nitro-3-aminopyridin entsprechen Beispiel 1. Ausbeute und Qualität des Reaktionsproduktes waren ebenfalls mit Beispiel 1 identisch.

B) Vergleichsbeispiele

Verschiedene 3-Aminopyridin-Derivate, hergestellt durch Einführung anderer Schutzgruppen für die Aminogruppe, wurden analog (Erfindungs-)Beispiel 1a nitriert. Dabei wurde bei folgenden Reaktionsbedingungen gearbeitet:
Reaktionstemperatur: ca. 30 - 80° C;
conc. $H_2SO_4$ (96 %ig);
Molverhältnis Ausgangsprodukt:$H_2SO_4$ = 1:(5-10);
$HNO_3$-Menge: 120 - 200 %, bezogen auf das Ausgangsprodukt.

Die Reaktionsprodukte wurden wie in (Erfindungs-)Beispiel 1b hydrolysiert. Die Ergebnisse sind in der folgenden Tabelle zusammengestellt:

| Schutzgruppe X | Verhalten der Pyridin-Derivate bei der Nitrierung in Schwefelsäure: Zersetzung | keine selektive Nitrierung in 2-Stellung |
|---|---|---|
| $-COCH_3$ | | + |
| $-COC_6H_5$ | | + |
| $-COC_6H_4-NO_2$(-m) | | + |
| $-COOCH_3$ | + | |
| $-COOC_6H_5$ | | + |

| Schutzgruppe X | Verhalten der Pyridin-Derivate bei der Nitrierung in Schwefelsäure | |
|---|---|---|
| | Zersetzung | keine selektive Nitrierung in 2-Stellung |
| $-CON(n-C_4H_9)_2$ | + | |
| $-CON(i-C_4H_9)_2$ | + | |
| $-CONH\ C_4H_9\ (n)$ | + | |
| $-CONH\ C_4H_9\ (i)$ | + | |
| $-SO_2C_6H_5$ | | + |
| $-CH_2-NH-$ 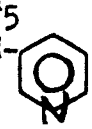 | + | |

Wie aus der Tabelle ersichtlich, werden die eingeführten Schutzgruppen unter Nitrierbedingungen entweder abgespalten, oder es erfolgt bei Stabilität der Schutzgruppe keine selektive Nitrierung in 2-Stellung des Pyridinrings.

**Patentansprüche**

1. Verfahren zur Herstellung von 2-Nitro-3-aminopyridin durch Nitrierung von 3-Aminopyridin, dessen Aminogruppe mit einer Schutzgruppe versehen ist, und anschließende hydrolytische Abspaltung der Schutzgruppe, dadurch gekennzeichnet, daß man als 3-Aminopyridin, dessen Aminogruppe mit einer Schutzgruppe versehen ist, N,N'-Di(3-pyridyl)-harnstoff verwendet.

2. Verfahren nach Anspruch 1, dadurch gekennzeichnet, daß die Nitrierung bei einer Temperatur von höchstens etwa 90° C, vorzugsweise bei etwa 50° bis 70° C durchgeführt wird.

3. Verfahren nach Anspruch 1 oder 2, dadurch gekennzeichnet, daß der N,N'-Di-(3-pyridyl)-harnstoff und $HNO_3$ in einem Mol-Verhältnis Pyridinring zu $HNO_3$ von 1 zu etwa 1 - 2, insbesondere 1 zu etwa 1,2 bis 1,4 eingesetzt wird.

4. Verfahren nach einem oder mehreren der Ansprüche 1 bis 3 dadurch gekennzeichnet, daß die Hydrolyse in überschüssigem Wasser vorgenommen wird, vorzugsweise unter Zusatz von polaren Lösungsmitteln, insbesondere von Alkoholen.

5. Verfahren nach Anspruch 4, dadurch gekennzeichnet, daß die Hydrolyse in einem wässrigen $C_1$- bis $C_4$-Alkohol mittels anorganischer Basen vorgenommen wird.

6. Verfahren nach einem oder mehreren der Ansprüche 1 bis 5, dadurch gekennzeichnet, daß die Nitrierung mittels Salpetersäure, zweckmäßig in Gegenwart von Schwefelsäure, insbesondere aber mit einem Gemisch aus Salpetersäure und Schwefelsäure durchgeführt wird.

7. N,N'-Di-(2-nitro-3-pyridyl)-harnstoff der Formel

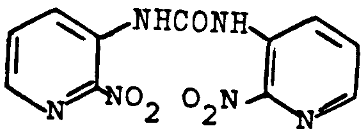

.

## Claims

1. A process for the preparation of 2-nitro-3-aminopyridine by nitrating 3-aminopyridine whose amino group is provided with a protective group, and subsequent hydrolytic elimination of the protective group, which comprises using N,N'-di(3-pyridyl)-urea as the 3-aminopyridine whose amino group is provided with a protective group.

2. The process as claimed in claim 1, wherein the nitration is carried out at a temperature of not more than approximately 90 ° C, preferably at approximately 50 ° to 70 ° C.

3. The process as claimed in claim 1 or 2, wherein N,N'-di-(3-pyridyl)-urea and $HNO_3$ are reacted in a molar ratio of pyridine ring to $HNO_3$ of 1 to approximately 1-2, in particular 1 to approximately 1.2 to 1.4.

4. The process as claimed in one or more of claims 1 to 3, wherein the hydrolysis is carried out in an excess of water, preferably with an addition of polar solvents, in particular alcohols.

5. The process as claimed in claim 4, wherein the hydrolysis is carried out in an aqueous $C_1$- to $C_4$-alcoholby means of inorganic bases.

6. The process as claimed in one or more of claims 1 to 5, wherein the nitration is carried out by means of nitric acid, expediently in the presence of sulfuric acid, but in particular using a mixture of nitric acid and sulfuric acid.

7. N,N'-Di-(2-nitro-3-pyridyl)-urea of the formula

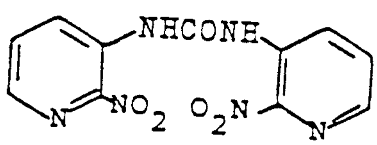

.

## Revendications

1. Procédé pour préparer la 2-nitro-3-amino-pyridine par nitration d'une 3-amino-pyridine dont le radical amino est muni d'un radical protecteur, puis coupure du radical protecteur par hydrolyse, procédé caractérisé en ce qu'on utilise, comme 3-amino-pyridine dont le radical amino porte un radical protecteur, la N,N'-bis-(3-pyridyl)-urée.

2. Procédé selon la revendicaiton 1 caractérisé en ce que la nitration est effectuée à une température d'au plus environ 90 ° C, de préférence à une température comprise entre environ 50 et 70 ° C.

3. Procédé selon l'une des revendications 1 et 2, caractérisé en ce que la N,N'-bis-(3-pyridyl)-urée et le $HNO_3$ sont mis en jeu en un rapport molaire du cycle pyridinique à $HNO_3$ compris entre environ 1:1 et 1:2, plus particulièrement entre environ 1:1,2 et 1:1,4.

4. Procédé selon une ou plusieurs des revendications 1 à 3, caractérisé en ce que l'hydrolyse est effectuée dans un excès d'eau, de préférence en présence de solvants polaires, plus particulièrement d'alcools.

5. Procédé selon la revendication 4 caractérisé en ce que l'hydrolyse est effectuée dans un alcol en $C_1$-$C_4$ aqueux, au moyen de bases inorganiques.

6. Procédé selon une ou plusieurs des revendications 1 à 5, caractérisé en ce que la nitration est effectuée au moyen d'acide nitrique, avantageusement en présence d'acide sulfurique, et plus particulièrement au moyen d'un mélange d'acide nitrique et d'acide sulfurique.

7. N,N'-Bis-(2-nitro-3-pyridyl)-urée de formule :